Europäisches Patentamt

European Patent Office

Office européen des brevets

(11) Publication number: **0 189 849**
A2

# (12) EUROPEAN PATENT APPLICATION

(21) Application number: 86100897.7

(22) Date of filing: 23.01.86

(51) Int. Cl.⁴: **A 61 K 39/395**
**A 61 K 45/06, A 61 K 43/00**

(30) Priority: 01.02.85 US 697264

(43) Date of publication of application:
06.08.86 Bulletin 86/32

(84) Designated Contracting States:
DE GB

(71) Applicant: Sloan-Kettering Institute For Cancer
Research
1275 York Avenue
New York New York 10021(US)

(72) Inventor: Houghton, Alan N.
450 E. Street
New York, N.Y. 10021(US)

(72) Inventor: Mintzer, David
169 Cedarbrook Road
Ardmore Pennsylvania 19003(US)

(72) Inventor: Cordon-Cardo, Carlos
450 E. 63 Street
New York, N.Y. 10021(US)

(72) Inventor: Welt, Sydney
504 East 81 Street
New York, N.Y. 10028(US)

(72) Inventor: Fliegel, Bettina L.
531 E. 87 Street, Apt. 1B
New York, N.Y. 10128(US)

(72) Inventor: Vadhan, Saroj
430 E. 67 Street
New York, N.Y. 10021(US)

(72) Inventor: Old, Lloyd J.
600 West End Avenue
New York, N.Y. 10024(US)

(72) Inventor: Carswell, Elizabeth
10 Otter Trail
Westport Connecticut 06880(US)

(72) Inventor: Melamed, Myron R.
35 Braeside Lane
Dobbs Ferry New York 10532(US)

(72) Inventor: Oettgen, Herbert F.
58 Overlook Drive
New Canaan Connecticut 06840(US)

(74) Representative: Patentanwälte Schulze Horn und
Hoffmeister
Goldstrasse 36
D-4400 Münster(DE)

(54) Method for treatment of neuroectodermal malignancies and epithelial carcinomas in humans.

(57) Major tumor regression with only mild side-effects is observed by treatment of human melanoma patients with $R_{24}$ mouse monoclonal antibody that identifies $GD_3$ ganglioside on the surface of melanoma and other cells of neuroectodermal origin. $R_{24}$ also serves to treat epithelial carcinomas as for example lung and/or breast tumor. Metasteses of the various cancers can also be treated.

EP 0 189 849 A2

This invention was supported in part by grants from the National Cancer Institute (CA-19267 and CA-08748). Therefore the United States Government has certain rights in this invention.

## Summary

This application concerns treatment of neuroectodermal disorders or cancers, espeically melanoma cancers in humans with mouse monoclonal antibody $R_{24}$, an $IgG_3$ antibody to $GD_3$ cell surface ganglioside restricted to certain cells of neuroectodermal origin.

## BACKGROUND

Mouse monoclonal antibodies have defined a large number of antigens on the surface of melanoma cells (Lloyd, K., (1983) R.B. Herberman, ed. IN: Basic and Clinical Tumor Immunology Martinus Nijoff Publ., Boston, M.A., U.S.A.; Reisfeld, R.A., Ferrone, S. (eds) (1982) Melanoma Antigens and Antibodies Plenum Press, New York). Although none of the antigens are melanoma-specific, some antigens have characteristics of differentiation antigens that mark melanocytes, melanoma and other cells of neuroectodermal origin (Houghton, A.N., et al. (1982) J. Exp. Med.

156:1755-1766). An IgG3 mouse monoclonal antibody, designated $R_{24}$, identifies one of the most restricted of these neuroectodermal markers. $R_{24}$ was generated by Dippold et al. (Dippold, W.G., et al. (1980) Proc. Nat'l. Acad. Sci., U.S.A. 77:6114-6118) (U.S. Patent application Serial No. 307,060) during a study of surface antigens of cultured melanoma cells and Pukel et al. (Pukel, C.S., et al. (1982) J. Exp. Med. 155:1133-1147) (U.S. Patent application S.N. 365,065) demonstrated that $R_{24}$ identifies the disialoganglioside $GD_3$. Both applications and papers are herein incorporated by reference. Analysis of cultured cells (Dippold, W.G., et al. (1980) Supra) and normal and malignant tissues (Real, F.X., et al. (1982) Proc. Am. Assoc. Cancer Res. 23:1006; Graus, F., et al. (1984) Brain Res. (in press)) showed that $R_{24}$ reacts with melanocytes, astrocytes, melanomas, astrocytomas, and a subset of sarcomas. $R_{24}$ also mediates a variety of biological effector functions, including tumor cell aggregation, human complement-mediated cytotoxicity, and antibody-dependent cell-mediated cytotoxicity with human effector cells (Dippold, W.G., et al. Cancer Res. 44:806-810; Knuth, A., et al. (1984) Proc. Am. Assoc. Cancer Res. 25:1005; Vogel, C-W, et al. (1983) Immunobiol. 164:309; Welt, S. et al., in preparation). $R_{24}$ is on deposit at the American Type Culture Collection, 1230 Parklawn Drive, Rockville MD. 20852 since November 29, 1983 and has the accession # HB8445).

Patients' tumors were shown to express $G_{D3}$ by indirect immunofluorescence tests on frozen sections prior to treatment. All patients had objectively measureable disease, a performance status (Karnofsky scale) of at least 60, and were off anticancer therapy for at least four weeks. No concurrent anticancer therapy was given during evaluation. Patients were considered evaluable six weeks after initiation of therapy; ten patients were evaluable and two patients had not yet reached the six week mark. The phase I trial with $R_{24}$ antibody was approved by the Institutional Review Board of Memorial Hospital; informed consent was obtained from all patients.

## DESCRIPTION

The examples shown serve to illustrate the invention without limiting it.

In the present study, we show the response of melanoma patients to $R_{24}$ with regard to different dose levels, toxicity, serological parameters and tumor response.

This work is described in a publication Houghton, A.N., et al. (1984) Proc. Nat'l. Acad. Sci. U.S.A. in press) which is hereby incorporated by reference.

## Preparation and Administration of $R_{24}$

$R_{24}$ was prepared from ascites of (BALB/C x 57 Bl)$F_1$ mice, purified by ammonium sulfate precipitation, chromatography over protein A Sepharose with pH 4.0 acetate buffer elution and further purification by G-25 Sephadex column with PBS pH 6.0 and filtered. Each $R_{24}$ batch was 3 mg/ml stored in 2% human serum albumin at -70°C. Each batch was tested for antibody reactivity and assayed for nucleic acids, 16 mouse viruses, bacteria, fungi and mycoplasma. Preparations underwent standard safety testing in mice and guinea pigs and pyrogenicity testing in rabbits.

$R_{24}$ was administered by intravenous infusion in 100-200 ml 0.9% saline and 5% human serum albumin. Skin tests with 0.1 micro g $R_{24}$ were done before the first treatment. The schedule of treatment was 1 mg/M$^2$ or 10 mg/M$^2$ every other day for eight treatments or 30 mg/M$^2$ per day by continuous in fusion on days 1 through 5 and 8 through 12.

### Serological Tests

$R_{24}$ antibody titers were determined by testing serum samples in protein A mixed adsorption assays (PA assays) (Pfreundschuh, M., et al. (1978) Proc. Nat'l. Acad. Sci., U.S.A. 75:5122-5126) against the melanoma target cell line SK-MEL-28. $R_{24}$ concentrations were measured by an enzyme-linked immunoassay. Falcon 3034 plates (Falcon Labware, Oxnard, CA, U.S.A) were precoated with purified $R_{24}$ 125 micrograms/ml. Rabbit antimouse IgG3 (Bionetics, Inc., Kensington, MD U.S.A.) diluted 1:100 was mixed (1:1 vol/vol) with patients' serum samples diluted 1:4 and incubated for 120 min. The mixture was transferred to the precoated wells, incubated for 60 min, and wells were washed with phosphate-buffered saline (PBS). Wells were incubated with goat antirabbit IgG linked to alkaline phosphatase (Sigma Chemical Co., St. Louse, MO, U.S.A.), for 60 min. Alkaline phosphatase activity was determined using p-nitrophenyldisodium phosphate substrate (Houghton, A.N., et al. (1983) J. Exp. Med. 158:58-65. $R_{24}$ concentrations were determined by comparison to standards using different concentrations of purified $R_{24}$ diluted in a pretreatment serum sample from the patient.

Human IgG antibody against mouse Ig was detected by enzyme-linked immunoassays. Falcon 3034 plates precoated with $R_{24}$ 50 micrograms/ml were incubated with patients' serum samples diluted 1:50 for 60 min, and washed with PBS. Antihuman IgG linked to alkaline phosphatase (Sigma Chemical Co.), was incubated in wells for 60 min, and reaction were measured by spectrophotometry (Houghton, A.N., et al. (1983) Supra).

Indirect immunofluorescence and immunoperoxidase procedures were performed as previously described in the art (Erlandson, R.A., et al. (1984) Am. J. Surg. Path. (in press)). The following reagents (Ortho Diagnostic Systems, Raritan, NJ, U.S.A.) were used for testing tissue sections: OKT-3, OKT-4, OKT-8 antibodies to T cell markers; OKB-2 and OKB-7 antibodies to B cell markers; OKM-1 and OKM-5 antibodies to macrophage markers; and OKIa-1 to human Ia antigens. Goat antisera to the human complement components C3, C5 and C9 were provided by Dr. Carl-Wilhelm Vogel. $R_{24}$ was used at concentration of 40 micrograms/ml. Mouse IgG in tumor tissues was detected by incubating section directly with biotinylated antimouse IgG, then with avidin-peroxidase conjugates and substrate. The toluidine blue staining method was used to detect tissue mast cells.

The examples below are for illustrative purposes and are not meant to limit the invention.

<u>Patient Characteristics</u>

Table I lists the clinical features of the 12 patients included in the study. The patients received total doses of 8 mg/m$^2$ (three patients), 80 mg/m$^2$ (six patients) and 240 mg/M$^2$ (three patients). The median age was 40 years (range 25-67) and the median performance status was 70 (range 60-90). Six patients had received prior chemotherapy, radiation therapy or interferon treatment. All patients had skin or soft tissue disease. In addition, visceral metastases were present in seven patients, including lung (three patients), brain (three patients) and liver (one patient).

Toxicity

No side-effects were observed in the three patients treated with the lowest does of $R_{24}$ (8 mg/M$^2$). All patients receiving a total dose of 80 mg/M$^2$ or greater had skin reactions, manifested by urticaria and pruritus usually developing 2-4 hrs after starting treatment. The intensity of skin reactions was related to the dose level and rate of antibody infusion. Urticaria characteristically appeared over tumor sites in the skin and subcutaneous soft tissue and around surgical scars where tumor had been removed. One patient (patient 6) developed urticarial lesions at sites where she had received melanoma cell vaccines/(ref) eight months previously. No reactions developed at $R_{24}$ skin test sites or around surgical scars unrelated to tumor treatment. Six patients went on to develop more generalized urticarial

0189849

lesions over the face, trunk or limbs (patients 5,7,8,10,11,12). Patient 5 experienced mild wheezing after rapid infusion of antibody (10 mg/hr); in this case the dose of $R_{24}$ was reduced to 66% of the total intended dose. Diphenhydramine was effective in controlling side-effects, but was only used for systemic symptoms.

At a dose level of 80 mg/M, the severity of skin reactions, particularly pruritus was found to be related to the rate of infusion of $R_{24}$. Treatment was tolerated well when the infusion rate was maintained at less than 5 mg/hr. At this rate, skin reactions usually occurred only after the first, second and third infusions and not after subsequent treatments. AT 240 mg/M$^2$, $R_{24}$ was administered by continuous infusion to maintain an infusion rate below 5 mg/hr. All three patients treated at this dose level developed urticaria initially restricted to tumor sites which later became generalized. Patients 10 and 11 experienced mild nausea and vomitting between 4 and 8 hrs after the start of treatment. Temperature elevation (up to 37.8°C) was seen in patients 11 and 12 near the end of treatment. No hepatic, renal, hematopoietic or neurological toxicity was observed and no changes were noted in vision or skin pigmentation over a period of up to 9 months follow-up.

## Antitumor Effects

Table I summarizes tumor responses in patients treated with $R_{24}$. Major tumor regression was observed as illustrated.

## Example 1

Patient 3, is a 36-year-old woman with primary malignant melanoma of the back, Clark's Level IV, 5mm depth of invasion diagnosed 9/82. In 8/83, the patient was found to have recurrent tumor and on 11/30/83, treatment with $R_{24}$ was started, 1 mg/$M^2$ (1.7 mg) every other day for eight doses. No toxicity or reactions at tumor sites were noticed during treatment. Sites of measurable disease included a firm 7 x 9 cm right axillary mass, a 4 x 2.5 cm subcutaneous nodule over the right hip and a 3 x 4.5 cm right paratracheal mass. A poorly defined density was present in the right upper lobe of the lung. Regression of tumor in the axilla and paratracheal region was first observed five weeks after starting treatment. The paratracheal mass has been undetectable since 3/84. The right axillary mass measured 1.2 x 0.8 cm in 5/84 and has continued to regress. The subcutaneous nodule over the right hip did not change in size but became very tender and inflamed by 2/5/84. Excisional biopsy of this lesion revealed hemorrhagic necrosis and inflammatory cell infiltrates with small nests of melanoma cells which stained weakly or not at all with $R_{24}$. By 5/84, the density in the right upper lung field had become better defined, and a needle biopsy revealed melanoma cells which reacted strongly with $R_{24}$.

## Example 2

Patient 4 had a malignant melanoma diagnosed 2/82, 7 mm thickness, Clark's Level IV, with tumor in 10 of 12 regional lymph nodes. By 1/84, the patient had extensive bulky skin and soft tissue metastases (over 100 lesions) on the left thigh, in the left and right inguinal areas, and on the lower abdomen, scrotum and penis. Treatment with $R_{24}$, 20 mg/$M^2$ every other day for eight doses, was started on 1/18/84. Two hours after the start of the first infusion, the patient developed severe pruritus and urticarial lesions around all tumor sites. Urticaria progressed to confluent erythrema over tumor sites and adjacent areas of the thigh, inguinal areas and lower abdominal wall. These reactions disappeared 18 hrs later. A milder reaction was seen after the second and third doses and no reactions were seen during subsequent treatments. Eight weeks following the end of $R_{24}$ treatment, there was enlargement of lesions in the right groin and left thigh and new lesions had appeared over the abdominal wall. However, four weeks later, all measurable lesions had decreased in size by greater than 50%. There has been continued tumor regression over the past six months and most sites are now tumor-free.


Example 3


Patient 10 is a 61-year-old man who developed a melanoma, 2.8 mm thickness, Clark's Level III, over the right scapular area in 1977. A solitary lesion of the left frontal lobe of the brain was detected in 1/82 and treated by left frontal lobe craniotomy followed by whole brain radiation therapy. Between 6/83 and 10/83, multiple subcutaneous tumors developed over the trunk, and the patient continued to progress during sequential treatment with dacarbazine, Pimozide, CCNU, and dibromodulcitol. Treatment with $R_{24}$, 240 mg/M$^2$, by intravenous infusion over two weeks was started on 6/11/84. The patient had more than 30 skin and soft tissue lesions on the trunk, extremities, scalp, face and neck measuring between 1 and 5 cm in diameter. Four hours after starting treatment the patient developed urticaria first around tumor lesions and then becoming generalized by six hours. The skin reactions abated over the next 12 hours and were gone by the fourth day of treatment. Regression (greater than 90%) of several pigmented tumors was seen at the end of $R_{24}$ treatment, and generalized regression of lesions was observed by four weeks after starting therapy. During tumor regression, subcutaenous ecchymoses were noted over six responding tumor sites. The patient achieved a partial remission (greater than 50% regression of measurable lesions) and regression of lesions has continued. In the face of regression of skin and soft tissue lesions, the patient developed left mild hemiplegia eight weeks after the start of therapy. CTT image of the brain demonstrated an enlarging lesion in the putamen with a necrotic center.

### Example 4

Mixed responses were observed in two other patients (patients 2 and 9) (Table I). Patient 7 and rapid progression of bulky skin and soft tissue disease with deterioration of performance status and received dacarbazine five weeks after the start of $R_{24}$ treatment; she achieved a partial response three weeks after dacarbazine treatment and has remained in remission for more than 20 weeks. Patient 5 also showed progression of disease with development of new skin nodules at six weeks. Treatment with dacarbazine resulted in a partial response lasting now more than 20 weeks.

<u>Serology</u>

Table II presents serological studies performed on patients treated with $R_{24}$ which show:

1.    There was heterogeneity in the expression of $G_{D3}$ in the melanoma specimens obtained before treatment, ranging from 40% positive cells to 100% positive cells.

2.    Peak $R_{24}$ levels were related to the amount of antibody received. Median peak $R_{24}$ levels were 0.8 microgram/ml at 8 mg/$M^2$, 7 microgram/ml at 80 mg/$M^2$, and 58 microgram/ml at 240 mg/$M^2$.

$R_{24}$ levels fell off rapidly after the last $R_{24}$ treatment and were usually less than 5% of peak levels by 18 hrs after the end of therapy.

3.    Elevated levels of human IgG against mouse Ig were detected in all evaluable patients between 15 and 40 days after the start of therapy.

4.    There was no evidence of antigenic modulation during therapy. Tumor cells biopsied or aspirated during therapy in patients 4 and 10 demonstrated continued expression of $G_{D3}$ in the face of substantial levels of circulating and tumor-bound $R_{24}$.

5. Progression of tumor after $R_{24}$ treatment was not related to outgrowth of $G_{D3}$ negative cells. Biopsies done after tumor persistence of progression showed strongly $G_{D3}$ positive melanoma cells in the lung lesion of patient 3 and skin lesions of patients 5, 6 and 9.

6. The amount of $R_{24}$ reaching tumor cells appeared to correspond to the dose level of antibody given. Mouse IgG was not detected in lesions from a patient treated at 8 mg/M$^2$ (patient 2), but was detected weakly around vessels in patient 4 (80 mg/M$^2$) and strongly around vessels and on a proportion of tumor cells in patient 10 (240 mg/M$^2$).

7. Inflammation at tumor sites involved several components associated with immune reactions. Tumors from patients 4 and 10 had increased numbers of mast cells with evidence of mast cell degranulation, evidence of complement deposition including C3, C5 and C9, and infiltration with T8$^+$/Ia$^+$ lymphocytes. Tumor tissue taken immediately before treatment did not demonstrate these charcteristics.

A variety of mechanisms may be involved in the antitumor activity of $R_{24}$, ranging from antibody-directed complement-mediated cytotoxicity and cell-mediated cytotoxicity, to tumor cell injury secondary to the inflammatory reaction elicited in the tumor bed by $R_{24}$. Tumor biopsies showed a range of inflammatory changes in $R_{24}$-treated patients, with infiltration of T cells and mast cells, mast cell degranulation, and deposition of C3, C5 and C9 complement components being among the most prominent. Dippold et al. have also observed inflammatory reactions over tumor sites in two patients treated with $R_{24}$ [Dippold, W.G., et al. Proc. Am. Assoc. Cancer Res. 25:978 (abstract]). However, no tumor regression was found.

In our studies of a large number of mouse monoclonal antibodies to surface antigens of human cancer, $R_{24}$ has been found to be unique in its ability to activate human complement to such a high degree and to induce extremely strong cell-mediated cytotoxicity. Whether these are general characteristics of antibodies (like $R_{24}$) that belong to the IgG3 subclass or are related to the nature of the G antigenic determinant is not known. These questions can be addressed by studying other classes and subclasses of $G_{D3}$ monoclonal antibodies and IgG3 antibodies to either surface antigens. Complement components, such as C5a, that are generated during complement activation are known to have inflammatogenic activity (Morgan, E.L., et al. 91984) Fed. Proc. 43:2543-2547.), and it is likely that these are

involved in $R_{24}$-directed inflammation at the tumor site. If complement activation plays a key role in $R_{24}$-induced inflammation and antitumor effects, attention needs to be directed at complement levels, both systemic and intratumoral, during $R_{24}$ therapy to determine whether complement availability might limit the therapeutic activity of $R_{24}$.

Another aspect of $R_{24}$ that needs clinical evaluation is the possibility that responses to chemotherapy may be enhanced or potentiated by $R_{24}$. Studies in nu/nu rats and mice have shown that the antitumor effect of $R_{24}$ is potentiated by drugs such as adriamycin. Increased tumor blood flow and altered susceptibility of tumor cells to the action of drugs as a consequence of antibody treatment are two possibilities that could account for increased drug sensitivity of $R_{24}$-treated animals. This observation may have its counterpart in the clear responses of two $R_{24}$-treated patients to dacarbazine. Thus $R_{24}$ could be combined with other chemotherapeutic anti-tumor agents selected but not limited to those such as the purine or pyrimidine analogues, the nitrosoureas, cisretinoic acid, pimozide, dibromodulcitol, DTIC, cytoxan, methotrexate hydroxyurea, 6-mercapto-purine, adriamycin, cisplatin, bleomycin, actinomycin D and/or radiological treatment such

as $^{60}Co$ or $^{90}yttrium$, $^{131}I$ or $^{211}astatine$. Natural agents or factors such as tumor necrosis factor (TNF) [Williamson et al (1983) Proc. nat'l. Acad. Sci USA 80:5397], BCG, autologous tumor cell vaccine [Houghton et al in Immunodermatology ed. by Bigan Safai and Robert A. Good Plenum, 1981 see p. 570], Interferon, Interleukin-2 (See Welte et al (1982) J. Exp. Med. 156:454) and U.S. Patent Application S.N. 603,580), Interleukin-1 and Lymphotoxins in general can also be used in conjunction with $R_{24}$, administered together or in linkage form. Some nitrosourea for example are BCNU [1,3-BIS(2-chloroethyl)-1-nitrosourea], CCNU [1-(2-chloroethyl)-3-cyclohexyl-1-nitrosourea and methyl CCNU. Purine and pyrimidine analogues may include 6-mercaptopurine, 5-fluorouracil, and 2,6 diaminapurine for example. Others will be apparant to those skilled in the art.

Also available for clinical use will be a link of the mouse variable region of $R_{24}$ to a human $F_c$ portion for a more human-like immunoglobulin. This can be done by linking the DNA region coding for the mouse variable portion of the Ig molecules with a DNA region coding for the $F_c$ portion of human immunoglobulin to produce a mixed $R_{24}$ with a human $F_c$ component which may be tolerated better at higher doses. It is also possible to link $R_{24}$ molecules to fluorescent agents to localize tumors.

Also these could be linked to $R_{24}$ to localize as well as treat the tumor in situ. $R_{24}$ has already been linked to $^{131}I$ to localize as well as treat tumors such as melanomas. Melanocyte disorders such as Nevi or pigmentation disorders could be treated as well. $R_{24}$ has also been found useful in treatment since it reacts with epithelial carcinomas such as those of lung and breast. The listing above is meant as a series of examples and is by no means limiting to the invention as described. Other examples will be apparent to those skilled in the art.

However, a dose-response relation to $R_{24}$ may not be straightforward, since patients' responses will be influenced by a number of other parameters, e.g., strength and heterogeneity of $G_{D3}$ antigen expression, tumor cell accessibility, and availability of accessory factors such as complement, histamine and inflammatory cells.

## TABLE I

CLINICAL FEATURES AND TUMOR RESPONSES OF PATIENTS
TREATED WITH $R_{24}$ MOUSE MONOCLONAL ANTIBODY

| | AGE | SEX | PRIMARY SITE | PERFORMANCE STATUS (KARNOFSKY SCALE) | PRIOR THERAPY | DOSE LEVEL (TOTAL DOSE) | SITES OF DISEASE | RESPONSE AND DURATION (BY SITE) |
|---|---|---|---|---|---|---|---|---|
| 1 | 55 | F | Foot | 90 | Intralesional | $8mg/M^2$ (16.8 mg) | Skin* | Progression after 4 weeks |
| 2 | 57 | F | Leg | 70 | Radiation therapy | $8mg/M^2$ (13.6 mg) | Skin* Brain | Partial response[1], 10 weeks Stable, 19 weeks |
| 3 | 36 | F | Trunk | 90 | BCG, Cisretinoic acid | $8mg/M^2$ (13.6 mg) | Axilla and Paratracheal Lymp Node Lung | Partial response[1], 38+ weeks<br><br>Progression after 24 weeks |
| 4 | 30 | M | Leg | 70 | Interferon, Intralesional therapy | $80mg/M^2$ (168 mg) | Skin and Lymph Nodes* | Partial response[1], 28+ weeks |
| 5 | 52 | M | Unknown | 70 | Intralesional therapy | $80mg/M^2$ (90 mg) | Skin and Lymph Nodes* | Progression after 6 weeks[2] |
| 6 | 25 | F | Neck | 70 | Autologous tumor cell vaccine | $80\ mg/M^2$ (144 mg) | Skin* Brain | Progression after 10 weeks |
| 7 | 44 | F | Leg | 70 | Intralesional therapy | $80\ mg/M^2$ (136 mg) | Skin and Nodes (axilla, retroperitoneum)* | Progression after 5 weeks[2] |
| 8 | 30 | M | Choroid | 60 | Methotrexate, PCNU, DTIC, Hydroxyurea | $80mg/M^2$ (144 mg) | Skin* Liver* | Progression died at 6 weeks |

## TABLE I  Cont'd.

### CLINICAL FEATURES AND TUMOR RESPONSES OF PATIENTS
### TREATED WITH $R_{24}$ MOUSE MONOCLONAL ANTIBODY

| | AGE | SEX | PRIMARY SITE | PERFORMANCE STATUS (KARNOFSKY SCALE) | PRIOR THERAPY | DOSE LEVEL (TOTAL DOSE) | SITES OF DISEASE | RESPONSE AND DURATION (BY SITE) |
|---|---|---|---|---|---|---|---|---|
| 9 | 67 | M | Trunk | 90 | DTIC, Interferon | $80mg/M^2$ (152 mg) | Skin* Lung* | Progression after 6 weeks Partial response[1], 10 weeks |
| 10 | 58 | M | Trunk | 70 | DTIC, CCNU, Dibromodulcitol, Pimozide | $240\ mg/M^2$ (528 mg) | Skin* | Partial response[1], $6^+$ weeks |
| 11 | 34 | F | Leg | 90 | DTIC, Actino-mycin D | $240mg/M^2$ (400 mg) | Retroperitoneal Lymph Nodes | Stable, $4^+$ weeks |
| 12 | 26 | F | Leg | 90 | Cytoxan | $240mg/M^2$ (400 mg) | Skin* Mediastinum* Lung | Stable, $4^+$ weeks Stable, $4^+$ weeks Stable, $4^+$ weeks |

*Sites of measurable disease.

[1]Partial response - greater than or equal to 50 reduction in the sum of the products of the maximum and perpendicular diameters of all measurable lesions for at least 4 weeks.

[2]Patients 5 and 7 subsequently achieved partial responses with dacarbazine treatment.

## TABLE II

### SEROLOGY AND IMMUNOPATHOLOGY OF PATIENTS TREATED WITH $R_{24}$

| PATIENT | $R_{24}$ REACTIVITY WITH MELANOMA BIOPSIES (PRETREATMENT) INTENSITY (0-3[+]) | % + Cells | $R_{24}$ SERUM LEVEL PEAK Day | Titer[1] | Mouse IgG3[2] | 18 HRS FOLLOWING THE END OF TREATMENT Titer | Mouse IgG3 | HUMAN IgG ANTI-MOUSE Ig POST/PRETREATMENT[3] (DAYS AFTER START OF TREATMENT) | IMMUNOPATHOLOGY (DAYS AFTER START OF TREATMENT) |
|---|---|---|---|---|---|---|---|---|---|
| 1 | 3[+] | 100% | 4 | 1/64 | 0.8 ug/ml | 1/8 | L.T.0.1 ug/ml | 4.1 (164 days) | Not examined |
| 2 | 3[+] | 80% | 7 | 1/32 | L.T. 0.1 ug/ml | 0 | L.T.0.1 ug/ml | 5.6 (70 days) | Increased infiltration with lymphocytes and mast cells. Frequent mast cell degranulation. No mouse IgG detected. (Day 13) |
| 3 | 2[+] | 70% | 9 | 1/128 | 2 ug/ml | 0 | L.T.0.1 ug/ml | 3.9 (100 days) | Increased infiltration with lymphocytes and mast cells. (Day 64) |
| 4. | 3[+] | 100% | 7 | 1/256 | 13 ug/ml | 1/32 | 0.5 ug/ml | 3.8 (80 days) | Increased infiltration with T8[+]/Ia[+]/T3[+]/T4[−]/B2[−]/B7[−]/M1[−]/M5[−] lymphocytes. Increase in the number of mast cells with frequent degranulation. Deposition of complement (C3,C5,C9). Mouse IgG in tumor biopsy primarily around vessels. (Day 13) |
| 5. | 2[+] | 100% | 7 | 1/256 | 3 ug/ml | 1/16 | L.T.0.1 ug/ml | 2.1 (40 days) | Increased infiltration with lymphocytes and mast cells. Frequent mast cell degranulation. (Day 32). |
| 6. | 2[+] | 70% | 7 | 1/128 | 7 ug/ml | 1/32 | L.T.0.1 ug/ml | 2.0 (40 days) | No inflammatory infiltrate. (Day 64). |
| 7. | 2[+] | 80% | 14 | 1/512 | 5 ug/ml | 1/128 | 1 ug/ml | 2.3 (40 days) | Not examined. |

## TABLE II Cont'd.

### SEROLOGY AND IMMUNOPATHOLOGY OF PATIENTS TREATED WITH $R_{24}$

| PATIENT | $R_{24}$ REACTIVITY WITH MELANOMA BIOPSIES (PRETREATMENT) INTENSITY (0-3$^+$) | % + Cells | $R_{24}$ SERUM LEVEL PEAK Day | Titer[1] | Mouse IgG$_3$[2] | 18 HRS FOLLOWING THE END OF TREATMENT Titer | Mouse IgG$_3$[2] | HUMAN IgG ANTI-MOUSE Ig POST/PRETREATMENT[3] (DAYS AFTER START OF TREATMENT) | IMMUNOPATHOLOGY (DAYS AFTER START OF TREATMENT) |
|---|---|---|---|---|---|---|---|---|---|
| 8. | 2$^+$ | 80% | 4 | 1/64 | 6 ug/ml | 0 | L.T.0.1 ug/ml | 1.3 (35 days) | Not examined. |
| 9. | 2$^+$ | 70% | 9 | 1/256 | 8 ug/ml | 0 | L.T.0.1 ug/ml | 3.3 (40 days) | Not examined. |
| 10. | 3$^+$ | 100% | 12 | 1/1024 | 58 ug/ml | 1/128 | (N.T.) | 1.8 (30 days) | Infiltration with T8$^+$/Ia$^+$/T3$^+$/ T4$^-$/B2$^-$/B7$^-$/M1$^-$/M5$^-$ lymphocytes. Increase in the number of mast cells with frequent degranulation. Deposition of complement (C3, C5, C9). Mouse IgG in tumor biopsy around vessels and in tumor. (Day 11). |
| 11. | 3$^+$ | 90% | 5 | 1/256 | 18 ug/ml | 1/128 | 1 ug/ml | 1.2 (20 days) | Not examined. |
| 12. | 2$^+$ | 40% | 5 | 1/800 | 62 ug/ml | 1/200 | 2 ug/ml | 1.3 (14 days) | Not examined |

[1] $R_{24}$ serum titers measured by PA mixed hemadsorption assay against SK-MEL-28 melanoma target cells.

[2] Mouse IgG$_3$ ($R_{24}$) levels in human serum determined by inhibition enzyme-linked immunoassay.

[3] Human IgG antimouse Ig determined by enzyme-linked immunoassay. Results are presented as the ratio of post-treatment: pre-treatment levels.

NT = not tested.
LT = less than.
ug = microgram

A1

What is Claimed:

1. Method for treatment of cancer of cells of neuroectodermal origin and epithelial cancers in humans which comprises

administering effective levels of purified $R_{24}$ mouse monoclonal antibody to a patient with a cancer of neuroectodermal origin and;

observing regression of the malignancy.

2. Method of Claim 1 wherein the epithelial cancers are lung or breast carcinoma.

3. Method for treatment of tumors of neuroectodermal origin according to the method of claim 1 wherein the neuroectodermal tumors are selected from the group consisting of malignant melanomas, astrocytomas, and neuroblastomas.

4. Method of Claim 3 wherein the malignant melanoma is metastatic.

5. Method of Claim 1 wherein $R_{24}$ is administered together with one or more other anti-tumor chemotherapeutic agents.

6.   Method of Claim 5 wherein $R_{24}$ is linked to the other chemotherapeutic agent.

7.   Method of Claim 5 wherein the other chemotherapeutic agent is selected from the group consisting of dacarbazine, adriamycin, pimozide, dibromodulcitol, bleomycin, actinomycin D, purine and pyrimidine analogues, nitrosoureas, cis-platin, radiation-emitting reagents, tumor necrosis factor, Interferons, Interleukin-2, Lymphotoxins, Interleukin-1, BCG, autologous tumor cell vaccine and mixtures thereof.

8.   Method of Claim 7 wherein the nitrosoureas are selected from the group consisting of CCNU, BCNU and methyl CCNU.

9.   Method of Claim 7 wherein the purine and pyrimidine analogues are selected from the group consisting of 6-mercaptopurine, 5-fluorouracil, 2,6 diaminopurine and mixtures thereof.

10.  Method of Claim 7 wherein the radiation emitting reagent is $^{60}Co$, $^{131}I$, or $^{90}yttrium$.

11.  Method for the treatment of melanocyte or pigmentation disorders according to the method of Claim 1.